(19) Europäisches Patentamt
European Patent Office
Office européen des brevets

(11) Numéro de publication : **0 125 195**
**B1**

(12) **FASCICULE DE BREVET EUROPÉEN**

(45) Date de publication du fascicule du brevet : **30.09.87**

(51) Int. Cl.$^4$ : **C 07 D311/30**, A 61 K 31/35

(21) Numéro de dépôt : **84450006.6**

(22) Date de dépôt : **07.03.84**

(54) **Nouveaux acides flavonecarboxyliques-4', leur méthode de préparation et leur application thérapeutique.**

(30) Priorité : **24.03.83 FR 8305013**

(43) Date de publication de la demande : **14.11.84 Bulletin 84/46**

(45) Mention de la délivrance du brevet : **30.09.87 Bulletin 87/40**

(84) Etats contractants désignés : **BE CH DE GB IT LI LU NL**

(56) Documents cités : **FR-A- 2 232 310**

(73) Titulaire : **SOCIETE CORTIAL S.A.**
**7 rue de l'Armorique**
**F-75015 Paris (FR)**

(72) Inventeur : **Creuzet, Marie-Hélène**
**Résidence Jardin de Gambetta T3**
**F-33000 Bordeaux (FR)**
Inventeur : **Feniou, Claude**
**5 rue du Général Guillomat**
**F-33600 Pessac (FR)**
Inventeur : **Guichard, Françoise**
**Les Cèdres Parc des Tourelles Rue St-Elisabeth**
**F-33200 Bordeaux (FR)**
Inventeur : **Mosser, Jacqueline**
**Rue du Colonel Rozanoff**
**F-33160 Saint-Médard-en-Jalles (FR)**
Inventeur : **Prat, Gisèle**
**Hameau de Noailles - Villa 18**
**F-33400 Talence (FR)**
Inventeur : **Pontagnier, Henri**
**21 rue Edouard Vaillant**
**F-33600 Pessac (FR)**

(74) Mandataire : **Tajan, Marie-Thérèse**
**LABORATOIRES SARGET Avenue du Président JF Kennedy**
**F-33701 Mérignac (FR)**

**EP 0 125 195 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne des acides flavonecarboxyliques-4′ ainsi que leurs dérivés pharmaceutiquement acceptables, leur méthode de préparation et leur application en thérapeutique.

Les composés faisant l'objet de la présente invention ont pour formule générale

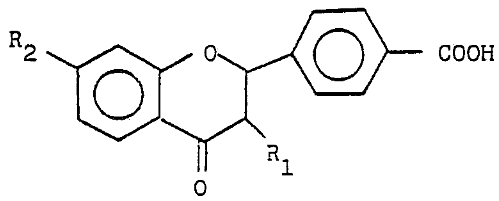

(I)

dans laquelle $R_1$ = H, OH, $OCOCH_3$, $OSO_2CH_3$, alkyle ramifié ou non contenant 1 à 5 atomes de carbone, $SO_2N(CH_3)_2$, $SO_2NHCH_3$, ◁; $SO_2NCH_3CH_2CH_2OH$, $SO_2NH_2$, $R_2$ = $NHCOR_3$, $NHCOCH_2NR_4R_5$, $NHSO_2CH_3$, $N(SO_2CH_3)_2$, avec $R_3$, $R_4$ et $R_5$ = alkyle inférieur contenant de 1 à 4 atomes de carbone ; $NR_4R_5$ peuvent également former un noyau pipéridine, pyrrolidine, morpholine ainsi que l'une des fonctions pharmaceutiquement acceptables suivantes dérivée de la fonction acide carboxylique à savoir les fonctions ester de méthyle, amide de pipéridine et les sels de métaux alcalins ou alcalinoterreux et ainsi que les sels d'additions d'acide minéraux ou organiques pharmaceutiquement acceptables.

Dans le cas où $R_2$ contient une fonction aminée, le composé peut être sous forme de sel d'acide minéral ou organique pharmaceutiquement acceptable.

On connaissait déjà des dérivés d'acide flavonecarboxylique-4′ et en particulier la Société Fisons avait déposé le 4 juin 1974 un brevet belge n° 815 896 décrivant notamment des acides flavonecarboxyliques-4′ substitués en position 7 sur le cycle benzopyranne par des radicaux H, halogène, alkyle, alcoxy, alcényle, amino, hydroxyle, trifluorométhyle, cyano, alkylamino, alcoxyalcoxy, hydroxyalcoxy ou nitro. Ces composés présentaient des activités antianaphylactiques.

Nous avons maintenant découvert que les acides flavonecarboxyliques-4′ substitués en position 7 par des groupes NHCO alkyle, $NHCOCH_2N$ , $NHSO_2CH_3$, $N(SO_2CH_3)_2$, $SO_2N(CH_3)_2$, $SO_2NHCH_3$, $SO_2NCH_3CH_2CH_2OH$ ou $SO_2NH_2$, composés chimiques, présentaient des propriétés inhibitrices de l'aldose réductase permettant leur emploi dans la prévention des complications, oculaires et nerveuses en particulier, du diabète. Ces composés sont également utiles en tant que diurétiques.

Les dérivés de formule (I) sont préparés de façon générale à partir des composés de formule

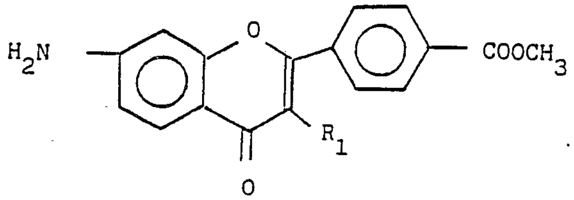

(II)

dans laquelle $R_1$ = H, OH, alkyle ramifié ou non contenant 1 à 5 atomes de carbone.

Le composé de formule (II) avec $R_1$ = OH est préparé de préférence par une réaction mettant en jeu l'acétamido-4 hydroxy-2 acétophénone et l'ester de méthyle de l'acide téréphtalaldéhydique conduisant à la chalcone de formule

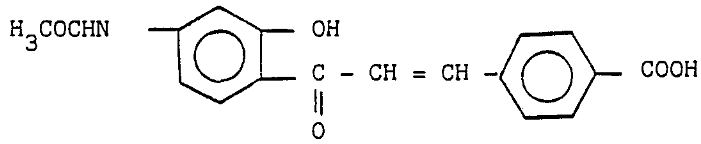

Cette chalcone subit une cyclisation oxydante dans l'éthanol en présence de potasse et d'eau oxygénée pour donner l'acide amino-7 hydroxy-3 flavonecarboxylique-4′ lui-même estérifié pour donner le composé de formule (II).

Le composé de formule (II) avec $R_1$ = H est préparé de préférence à partir de l'acétamido-4 hydroxy-2 acétophénone et du chlorure de l'acide paraméthoxycarbonylbenzoïque avec passage par un intermédiaire dicétonique cyclisé en présence d'acide chlorhydrique en milieu méthanolique.

Les composés de formule (II) avec $R_1$ = alkyle ramifié ou non contenant de 1 à 5 atomes de carbone sont préparés de préférence par une réaction entre un dérivé de formule

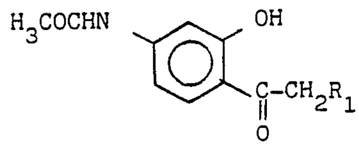

et le chlorure de l'acide paraméthoxycarbonylbenzoïque permettant d'obtenir directement les esters de méthyle des composés de formule (I) avec $R_1$ = alkyle ramifié ou non contenant de 1 à 5 atomes de carbone et $R_2 = NHCOCH_3$. La N-désacétylation du composé ainsi obtenu est réalisée de préférence dans le méthanol en présence d'acide chlorhydrique gazeux.

De façon plus générale, les esters de méthyle des composés de formule (I) avec $R_1$ = alkyle ramifié ou non contenant de 1 à 5 atomes de carbone et $R_2 = NHCOR_3$, $NHCOCH_2NR_4R_5$, $NHSO_2CH_3$, $N(SO_2CH_3)_2$, avec $R_3$, $R_4$ et $R_5$ = alkyle inférieur contenant de 1 à 4 atomes de carbone, et $NR_4R_5$ pouvant également former un noyau pipéridine, pyrrolidine, morpholine peuvent être obtenus directement par une réaction mettant en jeu un dérivé de formule

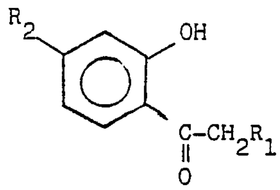

et le chlorure de l'acide paraméthoxycarbonylbenzoïque.

La fonction $NH_2$ des composés de formule générale (II) peut ensuite être, par les techniques classiques, transformée en groupement amide d'acide carboxylique ou en groupement sulfonamide. Cette fonction peut également être transformée en groupement chlorosulfonyl, lequel par réaction avec des amines conduit aux dérivés $R_2 = SO_2N(CH_3)_2$, $SO_2NHCH_3$, $SO_2NCH_3CH_2CH_2OH$, $SO_2NH_2$. Dans le cas où le dérivé (II) contient une fonction OH en $R_1$ il est possible de préparer par réaction avec un dérivé d'acide carboxylique ou sulfonique les esters de méthyle des dérivés de formule (I) avec $R_1 = OCOCH_3$ ou $OSO_2CH_3$. Les acides sont obtenus à partir des esters de méthyle par les méthodes classiques de la chimie.

La présente invention va être décrite de façon plus précise dans les exemples suivants.

## Exemple 1

Préparation de l'acide acétamido-7 flavonecarboxylique-4' ; composé de formule I avec $R_1$ = H, $R_2$ = — $NHCOCH_3$ ; nom de code COR19 85.

a) Préparation de l'ester de méthyle de l'acide amino-7 flavenocarboxylique-4'.

On chauffe sous agitation et au reflux de l'acétone pendant 10 heures le mélange constitué de 18 g d'acétamido-4 hydroxy-2 acétophénone, 1,7 l d'acétone anhydre, 100 g de carbonate de potassium anhydre, 22 g du chlorure de l'acide paraméthoxycarbonylbenzoïque. Après refroidissement et filtration, le filtrat est évaporé. De l'eau est ajoutée au résidu d'évaporation. Après acidification, le solide est filtré, lavé avec de l'eau et séché par azéotrope avec le benzène. Rendement 72 %. Le mélange constitué de 10 g de cette dicétone ainsi préparée, de 300 ml de méthanol et de 300 ml de méthanol saturé d'acide chlorhydrique gazeux est chauffé au reflux du méthanol pendant une heure. L'alcool est évaporé. De l'eau est rajoutée au résidu d'évaporation. La solution est alcalinisée et le précipité est filtré. Il est lavé à l'eau et séché par azéotrope avec du benzène. Rendement 65 %. PF supérieur à 260 °C.

b) Préparation de l'acétamido-7 flavonecarboxylate-4' de méthyle.

Le mélange constitué par 2 g d'ester de méthyle de l'acide amino-7 flavonecarboxylique-4' ainsi préparé, 30 cm³ de pyridine, 15 cm³ d'anhydride acétique est chauffé à 40 °C pendant 2 h et sous agitation. On vérifie que la réaction est terminée par chromatographie sur couches minces (solvant d'élution benzène (90) — dioxanne (25) — acide acétique (4). Le mélange réactionnel est versé dans 500 cm³ d'eau glacée. Le solide formé est filtré, lavé avec de l'eau et séché par formation d'azéotrope avec du benzène. Rendement 56 %.

c) Préparation de l'acide acétamido-7 flavonecarboxylique-4'.

6 g d'ester de méthyle de l'acide acétamido-7 flavonecarboxylique-4' sont dissous dans 600 cm³ de dioxanne. 12 g de carbonate de sodium dissous dans 250 cm³ d'eau sont ajoutés et le mélange est chauffé à 100 °C sous agitation, jusqu'à ce que la réaction soit totale, pendant environ 8 heures. La

réaction est contrôlée par chromatographie sur plaques (solvant d'élution benzène (90) — dioxanne (25) — acide acétique (4)).

Le mélange réactionnel est refroidi et versé dans un litre d'eau glacée. L'insoluble est filtré. Le filtrat est acidifié pour faire précipiter l'acide. Le précipité est filtré, lavé jusqu'à neutralité et séché par azéotrope avec du benzène. Rendement 53 %. PF non déterminable (décomposition à 240 °C). RMN dans le DMSOD6 : déplacements chimiques par rapport au TMS pris comme étalon interne : 2,2 ppm, 3H, singulet, $CH_3$ ; 7,0 ppm, 1H, singulet, H-3 ; 7,2-8,5 ppm, 7H, massif complexe, protons aromatiques ; 10,5 ppm, 2H, pic large, COOH + NH, échangeables avec $D_2O$.

## Exemple 2

Préparation du méthanesulfonamido-7 méthanesulfonyloxy-3 flavone carboxylate-4' de méthyle ; composé de formule I avec $R_1 = OSO_2Me$, $R_2 = NHSO_2Me$, ester de méthyle ; nom de code COR35 07.

a) Préparation de l'acide amino-7 hydroxy-3 flavonecarboxylique-4'.

38,6 g d'acétyl-2 acétamido-5 phénol sont dissous dans 600 cm$^3$ d'éthanol absolu. Le mélange est refroidi à 0 °C et agité sous argon. 400 cm$^3$ de potasse alcoolique à 25 % puis 39,2 g d'ester méthylique de l'acide téréphtalaldéhydique sont ajoutés. Le mélange est laissé sous agitation pendant 15 h à température ambiante sous argon. On rajoute de l'eau glacée et on acidifie par de l'acide acétique. On filtre puis on lave le solide avec de l'eau. La chalcone est séchée sous vide sur $P_2O_5$. Rendement 52 %. PF > 260 °C.

8 g de chalcone sont mis en suspension dans 320 ml d'éthanol. Le mélange est agité et chauffé à 40 °C. 60 ml de KOH N sont ajoutés. Le bain chauffant est retiré et 16 cm$^3$ d'eau oxygénée à 30 % sont ajoutés goutte à goutte. L'agitation est maintenue pendant une heure. 500 cm$^3$ d'eau glacée sont ajoutés. Le mélange est acidifié par de l'acide sulfurique dilué jusqu'à neutralité. Le solide (acide amino-7 hydroxy-3 flavone carboxylique-4') est filtré, lavé à l'eau et séché par azéotrope avec du benzène. Rendement 54 %. PF > 260 °C.

b) Préparation de l'amino-7 hydroxy-3 flavonecarboxylate-4' de méthyle.

Le mélange constitué par 4 g de l'acide précédemment préparé, 320 ml de méthanol, 8 ml d'acide méthanesulfonique est chauffé au reflux du méthanol sous agitation et pendant 15 heures. Le solvant est évaporé et le résidu d'évaporation est lavée à l'eau. L'ester de méthyle de l'acide est filtré et séché par azéotrope avec du benzène. Rendement 75 %.

c) Préparation du méthanesulfonamido-7 méthanesulfonyloxy-3 flavonecarboxylate-4' de méthyle.

2,5 g d'amino-7 hydroxy-3 flavonecarboxylate-4' de méthyle sont mis en suspension dans 25 cm$^3$ de benzène anhydre. Le mélange est refroidi à 0 °C et 10 cm$^3$ du chlorure de l'acide méthanesulfonique sont ajoutés goutte à goutte. Le mélange est mis sous agitation à température ambiante jusqu'à ce que la réaction soit totale. La réaction est contrôlée par chromatographie sur couches minces (solvant d'élution benzène (90) — dioxanne (25) — acide acétique (4)). Le mélange est versé dans 300 cm$^3$ d'eau glacée. Le précipité est filtré, lavé à l'eau et séché. Le composé ainsi obtenu est purifié par chromatographie sur colonne de silice ouverte. PF = 250 °C. RMN dans le DMSOD6 : 3,3 ppm, 3H, singulet, $CH_3$, méthanesulfonamido ; 3,6 ppm, 3H, singulet, $CH_3$ méthanesulfonyloxy ; 3,9 ppm, 3H, singulet, $CH_3$ ester de méthyle ; 7,1-8,4 ppm, 7H, massif complexe, protons aromatiques ; 10,7 ppm, 1H, dôme, NH, échangeable avec $D_2O$.

## Exemple 3

L'acide acétamido-7 acétoxy-3 flavonecarboxylique (composé de formule I avec $R_1 = OCOCH_3$, $R_2$ = NHCOCH3 ; nom de code COR19 94) est préparé selon l'exemple 2c par réaction de l'amino-7 hydroxy-3 flavonecarboxylate-4' de méthyle avec le chlorure de l'acide acétique puis saponification de l'ester de méthyle comme décrit dans l'exemple 1c.

PF > 300 °C. RMN dans DMFD7 + 5 gouttes de DMSOD6 : 2,2 ppm, 3H, singulet, $CH_3$ ; 2,4 ppm, 3H, singulet, $CH_3$ ; 7,3-8,4 ppm, 7H, massif complexe, protons aromatiques ; 10,6 ppm, 1H, pic large, NH, échangeable avec $D_2O$ ; 12,5 ppm, 1H, pic très étalé, COOH, échangeable avec $D_2O$.

## Exemple 4

Préparation de l'acide acétamido-7 propyl-3 flavonecarboxylique-4' ; composé de formule I avec $R_1 = C_3H_7$, $R_2 = NHCOCH_3$ ; nom de code COR19 91.

Le mélange constitué de 49,5 g de N-acétyl m-anisidine, 72,3 cm$^3$ de chlorure de l'acide valérique et 200 cm$^3$ de chlorure de méthylène est refroidi à 0 °C. 120 g de chlorure d'aluminium sont ajoutés petit à

petit. Le mélange réactionnel est porté au reflux pendant 2 heures sous agitation, refroidi puis versé dans l'eau glacée. Le composé huileux formé est extrait par du chloroforme. La phase chloroformique est extraite à son tour par une solution de soude 2N qui est ensuite acidifiée. On obtient ainsi 24 g d'acétamido-5 valéryl-2 phénol (PF 113 °C).

On maintient au reflux pendant 8 h sous agitation le mélange constitué par 23 g du phénol précédemment obtenu, 23 g du chlorure de l'acide paraméthoxycarbonylbenzoïque, 94 g de carbonate de potassium anhydre, 1,7 l d'acétone anhydre. Le mélange réactionnel est refroidi. Les sels minéraux sont éliminés par filtration et la phase acétonique est évaporée à sec. Le résidu est recristallisé dans le minimum d'éthanol. On obtient ainsi 7 g d'ester de méthyle de l'acide acétamido-7 propyl-3 flavonecarboxylique-4'. PF = 244 °C. Cet ester est saponifié selon la technique décrite dans l'exemple 1c pour obtenir l'acide COR19 91. PF > 300 °C. RMN dans DMSOD6 : 0,8 ppm, 3H, triplet, $CH_3$ (propyl) ; 1,1-1,8 ppm, 2H, multiplet mal résolu, $CH_2\underline{CH_2}CH_3$ ; 2,0-2,7 ppm, 5H, massif complexe, $CH_3$ (acétamido) + $\underline{CH_2}CH_2CH_3$ (dont $CH_3$ à 2,1 ppm) ; 7,2-8,3 ppm, 7H, massif complexe, protons aromatiques ; 10,4 ppm, 1H, pic large, NH, échangeable avec $D_2O$ ; 13,1 ppm, 1H, dôme, COOH, échangeable avec $D_2O$.

## Exemple 5

Préparation de l'acide acétamido-7 éthyl-3 flavonecarboxylique-4' ; composé de formule I avec $R_1 = C_2H_5$, $R_2 = NHCOCH_3$ ; nom de code COR19 93.

Ce composé est préparé selon la méthode décrite dans l'exemple 4. PF = 292 °C. RMN dans DMSOD6 : 1,1 ppm, 3H, triplet, $CH_3$ (éthyle) ; 2,0-2,7 ppm, 5H, massif complexe, $CH_3$ (acétamido) + $CH_2$ (dont $CH_3$ à 2,1 ppm) ; 7,3-8,3 ppm, 7H, massif complexe, protons aromatiques ; 10,6 ppm, 1H, pic large, NH, échangeable avec $D_2O$ ; 13,1 ppm, 1H, dôme, COOH, échangeable avec $D_2O$.

## Exemple 6

Préparation de l'amide de pipéridine de l'acide acétamido-7 propyl-3 flavonecarboxylique-4' ; composé de formule I avec $R_1 = C_3H_7$, $R_2 = NHCOCH_3$, amide de pipéridine ; nom de code COR35 05.

0,3 cm³ de $SOCl_2$ et 1 goutte de diméthylformamide sont ajoutés à 1 g d'acide acétamido-7 propyl-3 flavonecarboxylique-4', préparé selon l'exemple 4, dans 10 ml de dichloroéthane sec. Le mélange est maintenu au reflux pendant 3 h 30 mn puis laissé au repos pendant 15 h. Le chlorure de l'acide est filtré et lavé avec du dichloroéthane. Rendement 66 %.

4 g de pipéridine sont ajoutés à 0,7 g du chlorure d'acide. Le mélange est chauffé à 50-60 °C sous agitation pendant deux heures puis versé dans de l'eau glacée. L'amide est extrait par du chloroforme. La phase chloroformique est lavée à l'eau, séchée sur sulfate de sodium et évaporée. Le résidu est repris dans un peu d'éther éthylique, filtré et séché. Rendement 38 %. PF 238 °C. RMN dans $CDCL_3$ : 0,9 ppm, 3H, triplet, $CH_3$ (propyle) ; 1,3-2,0 ppm, 8H, massif complexe, $CH_2\underline{CH_2}CH_3$ + $3CH_2C$ de pipéridine ; 2,2 ppm, 3H, singulet, $CH_3$ (acétamido) ; 2,3-2,7 ppm, 2H, triplet mal résolu, $\underline{CH_2}CH_2CH_3$ ; 3,1-4,1 ppm, 4H, massif complexe, $CH_2N$ ; 7,1-8,3 ppm, 7H, massif complexe, protons aromatiques ; 9,8 ppm, 1H, pic large, NH, échangeable avec $D_2O$.

## Exemple 7

Préparation du méthanesulfonamido-7 propyl-3 flavonecarboxylate-4' de méthyle ; composé de formule I avec $R_1 = C_3H_7$, $R_2 = NHSO_2CH_3$, ester de méthyle ; nom de code COR19 99.

a) Préparation de l'amido-7 propyl-3 flavonecarboxylate-4' de méthyle.

14 g de l'acétamido-7 propyl-3 flavonecarboxylate-4' de méthyle préparé selon l'exemple 4 sont mis en suspension dans 700 cm³ de méthanol. Après addition de 300 cm³ de méthanol saturé d'acide chlorhydrique gazeux, le mélange est chauffé à 100 °C sous agitation pendant 2 heures. Le méthanol est évaporé et de l'eau est ajoutée au résidu. L'amino-7 propyl-3 flavonecarboxylate-4' de méthyl précipite. Le précipité est filtré, lavé à l'eau jusqu'à neutralité des eaux de lavage. On obtient ainsi 12 g de dérivé aminé.

b) Préparation du méthanesulfonamido-7 propyl-3 flavonecarboxylate-4' de méthyle.

3 g d'amine ainsi préparée sont dissous dans 15 cm³ de pyridine et additionnés goutte à goutte de 5 cm³ de chlorure de mésithyle. L'agitation est maintenue pendant 12 h à température ambiante puis le mélange réactionnel est versé dans de l'eau glacée. Le précipité formé est filtré, lavé et séché. On obtient ainsi 2 g de méthanesulfonamido-7 propyl-3 flavonecarboxylate-4' de méthyle. PF = 235 °C. RMN dans DMSOD6 : 0,8 ppm, 3H, triplet, $CH_3$ (propyle) ; 1,1-1,8 ppm, 2H, multiplet mal résolu, $CH_2\underline{CH_2}CH_3$ ; 2,2-2,6 ppm, 2H, triplet mal résolu, $\underline{CH_2}CH_2CH_3$ ; 3,2 ppm, 3H, singulet, $CH_3$ (méthanesulfonamido) ; 3,9 ppm, 3H, singulet, $CH_3$ ester ; 7,0-8,2 ppm ; 7H, massif complexe, protons aromatiques.

Exemple 8

Préparation de l'acide méthanesulfonamido-7 propyl-3 flavonecarboxylique-4' ; composé de formule I avec $R_1 = C_3H_7$, $R_2 = NHSO_2CH_3$ ; nom de code COR35 01.

2,7 g d'ester de méthyle préparé selon l'exemple 7 sont dissous dans 300 cm$^3$ de dioxanne. A cette solution est ajoutée une solution de 4 g de carbonate de sodium dans 80 cm$^3$ d'eau. Le mélange est porté au reflux sous agitation pendant 6 heures puis est refroidi et additionné d'eau glacée. L'insoluble est filtré puis le filtrat est acidifié par HCl concentré. Le précipité est filtré, lavé à l'eau et séché. On obtient ainsi 2 g d'acide. PF 272 °C. RMN dans DMSOD6 : 0,8 ppm, 3H, triplet, $CH_3$ (propyle) ; 1,1-1,8 ppm, 2H, multiplet mal résolu, $CH_2\underline{CH_2}CH_3$ ; 2,1-2,7 ppm, 2H, triplet mal résolu, $\underline{CH_2}CH_2CH_3$ ; 3,2 ppm, 3H, singulet, $CH_3$ (méthanesulfonamido) ; 7,1-8,3 ppm, 7H, massif complexe, protons aromatiques ; 10,7 ppm, 1H, dôme, NH échangeable avec $D_2O$ ; 13,2 ppm, 1H, dôme, COOH, échangeable avec $D_2O$.

Exemple 9

Synthèse du (morpholinoacétamido)-7 propyl-3 flavonecarboxylate-4' de méthyle. Composé de formule I avec $R_1 = C_3H_7$,

$R_2$ = NHCOCH$_2$ 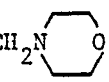 ,

ester de méthyle ; nom de code COR35 17.

On ajoute, sous agitation, 8 cm$^3$ de chlorure de chloroacétyle à un mélange constitué de 9,5 g d'amino-7 propyl-3 flavonecarboxylate-4' de méthyle fabriqué selon l'exemple 7a, 1 l de benzène et 9 g de $K_2CO_3$ anhydre. Le mélange est chauffé au reflux du benzène pendant 2 h puis refroidi. La phase benzénique est lavée à l'eau. Il se forme un précipité que l'on filtre et que l'on dissout dans du chloroforme. L'ensemble des phases benzénique et chloroformique est évaporé. Le (chloro-2 acétamido)-7 propyl-3 flavonecarboxylate-4' de méthyle est obtenu avec un redement de 80 %. PF = 162 °C.

Le mélange constitué par 9,2 g de ce dérivé, 700 cm$^3$ de benzène, 20 cm$^3$ de morpholine est chauffé au reflux du benzène sous agitation pendant 4 heures, puis refroidi. La phase benzénique est lavée à l'eau plusieurs fois, séchée sur sulfate de sodium et évaporée. Le résidu est recristallisé dans l'éthanol. Rendement 60 %. PF = 196 °C. RMN dans $CDCL_3$ : 0,9 ppm, 3H, triplet, $CH_3$ (propyle), 1,2-1,9 ppm, 2H, multiplet mal résolu, $CH_2\underline{CH_2}CH_3$ ; 2,3-2,9 ppm, 6H, massif complexe, $\underline{CH_2}CH_2CH_3$ + 2 $CH_2N$ (morpholine) ; 3,2 ppm, 2H, singulet, $COCH_2N$ ; 4,0 ppm, 3H, singulet, $CH_3$ ester ; 7,0-8,3 ppm, 7H, massif complexe, protons aromatiques ; 9,4 ppm, 1H, pic large, NH, échangeable avec $D_2O$.

Exemple 10

Synthèse du chlorhydrate de l'acide morpholinoacétamido-7 propyl-3 flavonecarboxylique-4' ; composé de formule I avec $R_1 = C_3H_7$,

$R_2$ = NHCOCH$_2$ 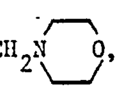 ,

chlorhydrate ; nom du code COR35 02.

On ajoute à 2 g d'ester de méthyle préparés selon l'exemple 9 dissous dans 250 cm$^3$ d'éthanol et 125 cm$^3$ de dioxanne, 2 g de carbonate de sodium dissous dans 60 cm$^3$ d'eau. Le mélange est maintenu au reflux sous vive agitation jusqu'à ce que la saponification soit totale puis refroidi et neutralisé par de l'acide acétique. Le précipité est filtré et recristallisé dans l'éthanol. Le composé est dissous dans du méthanol ; on ajoute quelques ml de méthanol saturé par HCl gazeux puis de l'éther éthylique pour faire précipiter le COR35 02. Le précipité est filtré et lavé par de l'éther éthylique. Rendement 40 %. RMN dans DMSOD6 : 0,8 ppm, 3H, triplet mal résolu, $CH_3$ (propyle) ; 1,1-1,8 ppm, 2H, multiplet mal résolu, $CH_2\underline{CH_2}CH_3$ ; 2,1-2,7 ppm, 2H, triplet mal résolu, $\underline{CH_2}CH_2CH_3$ ; 3,1-4,7 ppm, 10H, massif complexe, $NCH_2 + OCH_2$ ; 7,4-8,3 ppm, 7H, massif complexe, protons aromatiques ; 9-13 ppm, 3H, pic très étalé, NH + $NH^+$ + COOH, échangeables avec $D_2O$.

Exemple 11

Préparation du chlorhydrate de l'acide pipéridinoacétamido-7 propyl-3 flavonecarboxylique-4' ; composé de formule I avec $R_1 = C_3H_7$,

$R_2$ = NHCOCH$_2$ 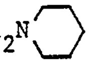

chlorhydrate ; nom de code COR19 95.

Le COR19 95 est préparé selon les techniques décrites dans les exemples 9 et 10. PF = 179 °C. RMN dans DMFD7 : 0,8 ppm, 3H, triplet, $CH_3$ (propyle) ; 1,2-2,7 ppm, 10H, massif complexe, $5CH_2C$ ; 3,1-3,8 ppm, 4H, massif complexe, $2N\text{-}CH_2$ (pipéridine) ; 4,4 ppm, 2H, singulet, $NCH_2CO$ ; 7,4-8,4 ppm, 7H, massif complexe, protons aromatiques ; 12,1 et 6-10 ppm, 3H, pic large et pic étalé, NH + $NH^+$ + COOH, échangeables avec $D_2O$.

Exemple 12

Préparation du N,N-diméthylsulfamoyl-7 propyl-3 flavonecarboxylate-4′ de méthyle ; composé de formule I avec $R_1 = C_3H_7$, $R_2 = SO_2N(CH_3)_2$ ; ester de méthyle ; nom de code COR35 08.

a) Préparation du chlorosulfonyl-7 propyl-3 flavonecarboxylate-4′ de méthyle.

6 g d'amino-7 propyl-3 flavonecarboxylate-4′ de méthyle sont mis en suspension sous agitation dans 120 $cm^3$ d'acide acétique. On ajoute 18 $cm^3$ d'acide chlorhydrique concentré en 5-10 mn, puis goutte à goutte une solution de 4,5 g de nitrite de sodium dans 6 $cm^6$ d'eau en maintenant la température de la réaction à 5 °C. Le mélange est maintenu à température ambiante pendant 20 mn puis versé par fraction dans une solution glacée de 80 $cm^3$ d'acide acétique saturée de $SO_2$ (solution à laquelle ont été préalablement ajoutés 4 g de $CuCl_2 \cdot 2H_2O$ dissous dans 8 $cm^3$ d'eau). L'agitation est maintenue à la température ambiante pendant 2 h, puis 400 $cm^3$ d'eau glacée sont additionnés. Le précipité formé est filtré, lavé et séché. On obtient ainsi 4,8 g de chlorosulfonyl-7 propyl-3 flavonecarboxylate-4′ de méthyle. PF = 128 °C.

b) Préparation du N,N-diméthylsulfamoyl-7 propyl-3 flavonecarboxylate-4′ de méthyle.

1,2 g du dérivé chlorosulfonyl-7 sont dissous dans 100 $cm^3$ de benzène. Après addition de 10 $cm^3$ de diméthylamine, le mélange est porté au reflux et maintenu pendant deux heures sous agitation. Après refroidissement, on ajoute 100 $cm^3$ d'éther éthylique et la phase organique est lavée à l'eau. Cette phase est ensuite séchée sur sulfate de sodium et évaporée. Le résidu solide obtenu est recristallisé dans l'éthanol. On obtient ainsi 0,7 g de COR35 08 ; PF = 166 °C. RMN dans $CDCl_3$ : 0,9 ppm, 3H, triplet, $CH_3$ (propyle) ; 1,2-1,9 ppm, 2H, multiplet mal résolu, $CH_2\underline{CH_2}CH_3$ ; 2,4-2,7 ppm, 2H, triplet mal résolu, $\underline{CH_2}CH_2CH_3$ ; 2,8 ppm, 6H, singulet, $NCH_3$ ; 4,0 ppm, 3H, singulet, $CH_3$ ester ; 7,6-8,6 ppm, 7H, massif complexe, protons aromatiques.

Exemple 13

Préparation de l'acide N,N-diméthylsulfamoyl-7 propyl-3 flavonecarboxylique-4′ ; composé de formule I avec $R_1 = C_3H_7$, $R_2 = SO_2N(CH_3)_2$ ; nom de code COR35 09.

2 g de l'ester de méthyle préparés selon l'exemple 12 sont dissous dans 100 $cm^3$ de dioxanne puis additionnés d'une solution de 2 g de carbonate de sodium dissous dans 40 $cm^3$ d'eau. Le mélange est maintenu au reflux sous agitation pendant 5 h puis refroidi et additionné de 300 $cm^3$ d'eau glacée. L'insoluble est filtré, le filtrat est acidifié par HCl concentré puis le précipité formé est filtré et séché. On obtient ainsi 1 g de COR35 09. PF = 271 °C. RMN dans DMSOD6 : 0,8 ppm, 3H, triplet, $CH_3$ (propyle) ; 1,1-1,8 ppm, 2H, triplet mal résolu, $CH_2\underline{CH_2}CH_3$ ; 2,2-2,6 ppm, 2H, triplet mal résolu, $\underline{CH_2}CH_2CH_3$ ; 2,7 ppm, 6H, singulet, $NCH_3$ ; 7,7-8,4 ppm, 7H, massif complexe, protons aromatiques ; 13,3 ppm, 1H, dôme, COOH, échangeable avec $D_2O$.

Exemple 14

Préparation du N-méthylsulfamoyl-7 propyl-3 flavone-carboxylate-4′ de méthyle ; composé de formule I avec $R_1 = C_3H_7$, $R_2 = SO_2NHCH_3$, ester de méthyle ; nom de code COR35 10.

Ce composé est préparé selon l'exemple 12b à partir de chlorosulfonyl-7 propyl-3 flavonecarboxylate-4′ de méthyle et de méthylamine. PF = 134 °C. RMN dans $CDCl_3$ : 0,9 ppm, 3H, triplet, $CH_3$ (propyle) ; 1,2-1,9 ppm, 2H, multiplet mal résolu, $CH_2\underline{CH_2}CH_3$ ; 2,3-2,6 ppm, 2H, triplet mal résolu, $\underline{CH_2}CH_2CH_3$ ; 2,8 ppm, 3H, doublet, $NCH_3$ ; 4,0 ppm, 3H, singulet, $CH_3$ ester ; 5,5 ppm, 1H, quadruplet, NH échangeable avec $D_2O$ ; 7,7-8,5 ppm, 7H, massif complexe, protons aromatiques.

Exemple 15

Préparation de l'acide N-méthylsulfamoyl-7 propyl-3 flavonecarboxylique-4′ ; composé de formule I avec $R_1 = C_3H_7$ ; $R_2 = SO_2NHCH_3$ ; nom de code COR35 11.

Ce composé est préparé par saponification selon l'exemple 13 de l'ester de méthyle obtenu selon l'exemple 14. PF = 261 °C. RMN dans DMFD7 : 0,9 ppm, 3H, triplet, $CH_3$ (propyle) ; 1,2-2,0 ppm, 2H,

multiplet mal résolu, $CH_2CH_2CH_3$ ; 2,3-2,9 ppm, 5H, massif complexe, $CH_2CH_2CH_3 + NCH_3$ ; 7,7-8,5 ppm, 7H, massif complexe, protons aromatiques ; 7-10 ppm, 2H, pic très étalé, NH + COOH, échangeables avec $D_2O$.

Exemple 16

Préparation de l'acide (N-hydroxyéthyl N-méthyl sulfamoyl)-7 propyl-3 flavonecarboxylique-4' ; composé de formule I avec $R_1 = C_3H_7$, $R_2 = SO_2NCH_2CH_2CH_2OH$ ; nom de code COR35 19.

L'ester de méthyle de cet acide est préparé à partir de chlorosulfonyl-7 propyl-3 flavonecarboxylate-4' de méthyle et de (méthylamino)-2 éthanol selon l'exemple 12b ; PF = 90-95 °C. L'acide est préparé à partir de cet ester par saponification selon l'exemple 13.

PF = 191 °C. RMN dans DMFD7 : 0,9 ppm, 3H, triplet, $CH_3$ (propyle) ; 1,2-2,0 ppm ; multiplet mal résolu, $CH_2CH_2CH_3$ ; 2,3-2,7 ppm, 2H, triplet mal résolu, $CH_2CH_2CH_3$ ; 3,0 ppm, 3H, singulet, $NCH_3$ ; 3,3 ppm, 2H, triplet, $NCH_2$ ; 3,7 ppm, 2H, triplet, $CH_2O$ ; 7,7-8,4 ppm, 7H, massif complexe, protons aromatiques ; 5-10 ppm, 2H, pic très étalé, OH + C00H, échangeables avec $D_2O$.

Exemple 17

Préparation d'acide sulfamoyl-7 propyl-3 flavonecarboxylique-4' ; composé de formule I avec $R_1 = C_3H_7$, $R_2 = SO_2NH_2$ ; nom de code COR35 22.

5 g de chlorosulfonyl-7 propyl-3 flavonecarboxylate-4' de méthyle préparé selon l'exemple 12a sont dissous dans 300 $cm^3$ de benzène. On fait passer un courant d'ammoniac dans la solution pendant 10 mn. Le précipité est filtré et lavé avec du benzène. Rendement 68 %.

3,2 g de sulfamoyl-7 propyl-3 flavonecarboxylate-4' de méthyle ainsi préparé sont dissous dans 200 $cm^3$ de dioxanne. 3 g de carbonate de sodium dissous dans 150 $cm^3$ d'eau sont ajoutés. Le mélange est maintenu sous forte agitation pendant 4 h au reflux du dioxanne. On vérifie que la réaction est totale en chromatographie sur couches minces. On rajoute de l'eau glacée au mélange refroidi et on filtre sur papier plissé. Le mélange est acidifié. Le précipité est filtré puis dissous dans l'éthanol bouillant. La solution est filtrée sur filtre millipores et le filtrat est concentré pour faire reprécipiter l'acide qui est filtré après refroidissement. Rendement 55 %. PF = 287 °C. RMN dans DMSOD6 : 0,8 ppm, 3H triplet, $CH_3$ (propyle) ; 1,1-1,9 ppm, 2H, multiplet mal résolu, $CH_2CH_2CH_3$ ; 2,2-2,7 ppm, 2H, triplet mal résolu, $CH_2CH_2CH_3$ ; 7,5-8,4 ppm, 9H, massif complexe, $NH_2$ + protons aromatiques ; 13,3 ppm, 1H, dôme, COOH, échangeable avec $D_2O$.

Exemple 18

Préparation du chlorhydrate de l'acide (pipéridinoacétamido)-7 flavonecarboxylique-4' ; composé de formule I avec $R_1$ = H,

$R_2$ = $NHCOCH_2$ 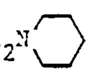 ,

chlorhydrate ; nom de code COR35 03.

L'ester de méthyle de cet acide est préparé à partir de l'amino-7 flavonecarboxylate-4' de méthyle, selon l'exemple 9, puis saponifié et transformé en chlorhydrate selon l'exemple 10. PF = 299 °C.

Les propriétés pharmacologiques des composés faisant l'objet de la présente invention sont exposées ci-après.

L'intérêt des composés de la présente invention dans le traitement et la prophylaxie des complications du diabète a été mis en évidence in vitro par la détermination de l'activité inhibitrice de l'aldose réductase et in vivo dans les modèles de la neuropathie induite par la streptozotocine et de la cataracte induite par le galactose.

Inhibition de l'aldose réductase in vitro

L'enzyme est extraite de cristallins de bœufs par la méthode décrite par Hayman S. et Kinoshita J.H. (J. Biol. Chem., 1965, 240, 2, 877). Le pourcentage d'inhibition de la capacité de l'enzyme à réduire la glycéraldéhyde en glycérol sous l'effet du composé à tester est déterminé par dosage spectrophotométrique de la quantité de NADPH réagissant suivant la méthode décrite par Hayman et Kinoshita. Nous donnons ci-après entre parenthèse après chaque composé la valeur du log 1/C150 où C150 représente la concentration exprimée en mole/1 entraînant 50 % d'inhibition de l'activité enzymatique : COR19 85 (4,9) ; COR19 91 (5,5) ; COR19 93 (5,1) ; COR19 94 (5,2) ; COR19 99 (5,4) ; COR35 01 (6,9) ; COR35 02 (5,15) ; COR35 07 (6,3) ; COR35 09 (5,7) ; COR35 11 (5,9) ; COR35 19 (5,8) ; COR35 22 (5,2).

Neuropathie à la streptozotocine

4 lots de 10 rats pesant environ 200 g sont traités de J — 2 à J + 3 par un julep gommeux du composé à tester. A J0 les animaux reçoivent une injection ip de 100 mg/kg de streptozotocine dissoute dans un tampon citrate. La glycémie des rats est déterminée 24 h et 3 jours après l'injection de l'agent diabétogène. Les rats sont sacrifiés le 3ᵉ jour après l'injection de streptozotocine et les nerfs sciatiques sont prélevés pour déterminer les taux de sorbitol et d'inositol. Testé dans ces conditions, COR35 01 entraîne à la dose de 100 mg/kg/j une diminution du taux de sorbitol (taux de 0,983 ± 0,334 mg/kg chez les animaux témoins et de 0,426 ± 0,171 chez les animaux traités) et d'inositol (taux égal à 1,655 ± 0,372 mg/kg chez les animaux témoins et à 1,184 ± 0,340 chez les animaux traités) dans les nerfs sciatiques. Le COR35 01 n'a pas d'effet sur la glycémie des animaux. Celle-ci a pour valeur 4,03 ± 0,89 (J1) et 5,44 ± 0,90 (J2) chez les animaux témoins et 4,07 ± 1,03 (J1) et 6,30 ± 0,97 (J2) chez les animaux traités.

Cataractes au galactose

Des rats âgés de 14 jours sont stabulés par cage de 11 avec une mère durant 3 jours. Du 4° au 9° jour les animaux reçoivent le composé à tester ou le composé de référence. A la fin du prétraitement, les mères sont retirées. Sur 11 animaux, 2 sont éliminés de façon à obtenir des lots de poids homogènes. Les rats sélectionnés sont répartis en cages 3 par 3, par randomisation et le régime contenant 20 % de galactose est mis en place. Pendant 14 jours les cristallins sont observés à l'ophtalmoscope. Le degré de cataracte est apprécié selon un système de cotation allant de 0 à 3.

Les cotations attribuées aux animaux traités sont comparées à celles des témoins par le test U de Mann et Whitney (Schwartz D. Méthodes statistiques à l'usage des médecins et des biologistes, Ed Flammarion, Paris, 1963). Les résultats sont représentés dans le tableau ci-après.

La toxicité des composés de la présente invention est déterminée chez la souris. Ainsi les DL 0 du COR19 91, du COR19 94 et du COR35 01 sont supérieures à 2 000 mg/kg pour la voie orale. Le COR35 17 provoque, après administration orale d'une dose de 1 000 mg/kg, 10 % de mortalité. Par voie intrapéritonéale le COR35 01 n'entraîne pas de mortalité à 250 mg/kg et entraîne 20 % de mortalité à 500 mg/kg.

A côté de leur intérêt dans la thérapeutique curative et préventive des complications du diabète les composés faisant l'objet de la présente invention sont également utiles en tant que diurétiques. Ainsi le composé COR35 07 testé chez des rats selon la méthode de Lipschitz et coll. (J. Pharm. Exp. Therap., 1943, 79, 97-110) entraîne une augmentation de l'excrétion urinaire de sodium (la natriurie est multipliée par 2,6 après administration orale de 20 mg/kg).

Compte tenu de leurs propriétés jointes à une faible toxicité, les composés selon la présente invention sont utiles en thérapeutique humaine et vétérinaire, par exemple dans le traitement et la prévention des cataractes métaboliques et en particulier diabétiques, dans le traitement et la prévention des neuropathies diabétiques, dans le traitement des œdèmes et des rétentions hydrosodées, dans le traitement de l'hypertension artérielle. Les composés faisant l'objet de la présente invention peuvent être utilisés seuls ou associés à des antihypertenseurs ou à des antidiabétiques. Ils seront administrés, associés aux véhicules et excipients appropriés, par voie orale sous forme de dragées, comprimés, sirop, ampoules buvables, par voie rectale sous forme de suppositoires, par voie parentérale en injections sous cutanées, intramusculaires, intraveineuses, par voie topique sous forme de pommades ou de gels. Ils peuvent également entrer dans des compositions à usage ophtalmique sous forme de collyres ou pommades. Les doses administrées varieront selon l'indication et le sujet de 5 à 500 mg/j en 2 à 6 prises pour la voie orale, de 5 à 500 mg/j en 1 ou 2 prises pour la voie rectale, de 0,5 à 50 mg par injection pour les voies parentérales.

(Voir tableau page 10)

Cataracte au galactose indice moyen

| Traitement | J + 1 | J + 2 | J + 3 | J + 4 | J + 5 | J + 6 | J + 7 | J + 8 | J + 9 | J + 10 | J + 11 | J + 12 | J + 13 |
|---|---|---|---|---|---|---|---|---|---|---|---|---|---|
| Témoins I gomme | 0,0 ±0,00 | 0,0 ±0,0 | 0,41 ±0,38 | 1,13 ±0,44 | 1,88 ±0,64 | 2,75 ±0,46 | 2,81 ±0,37 | 2,75 ±0,38 | 2,81 ±0,26 | 2,75 ±0,38 | 2;63 ±0,44 | 2,63 ±0,35 | 2,19 ±0,46 |
| COR35 09 100mg/kg/j | 0;0 ±0,00 | 0,0 ±0,0 | 0,031 ±0,09 | 0,47 ±0,34 S | 0,97 ±0,43 S | 1,44 ±0,62 S | 1,69 ±0,65 S | 1,81 ±0,59 S | 2,06 ±0,73 S | 1,88 ±0,69 S | 1,69 ±0,53 S | 1,81 ±0,37 S | 1,50 ±0,46 S |

**Revendications**

1. Composés de formule générale

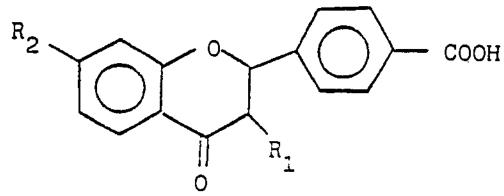

dans laquelle $R_1$ = H, OH, $OCOCH_3$, $OSO_2CH_3$, alkyle ramifié ou non contenant 1 à 5 atomes de carbone, $R_2$ = $NHCOR_3$, $NHCOCH_2NR_4R_5$, $NHSO_2CH_3$, $N(SO_2CH_3)_2$, $SO_2N(CH_3)_2$, $SO_2NHCH_3$, $SO_2NCH_3CH_2CH_2OH$, $SO_2NH_2$ avec $R_3$, $R_4$ et $R_5$ = alkyle inférieur contenant de 1 à 4 atomes de carbone, $NR_4R_5$ pouvant également former un noyau pipéridine, pyrrolidine, morpholine, ainsi que l'une des fonctions pharmaceutiquement acceptables suivantes dérivée de la fonction acide carboxylique à savoir les fonctions ester de méthyle, amide de pipéridine et les sels de métaux alcalins ou alcalinoterreux et ainsi que les sels d'additions d'acide minéraux ou organiques pharmaceutiquement acceptables.

2. Procédé de préparation des esters de méthyle des composés selon la revendication 1 dans lesquels $R_1$ = alkyle ramifié ou non contenant de 1 à 5 atomes de carbone et $R_2$ = $NHCOR_3$, $NHCOCH_2NR_4R_5$, $NHSO_2CH_3$, $N(SO_2CH_3)_2$, avec $R_3$, $R_4$ et $R_5$ = alkyle inférieur contenant de 1 à 4 atomes de carbone ou $NR_4R_5$ pouvant également former un noyau pipéridine, pyrrolidine, morpholine, par une réaction mettant en jeu un dérivé de formule

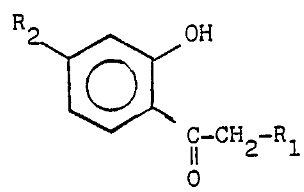

et le chlorure de l'acide paraméthoxycarbonylbenzoïque.

3. Procédé de préparation selon la revendication 2 des esters de méthyle de composés selon la revendication 1 dans lesquels $R_1$ = alkyle ramifié ou non contenant de 1 à 5 atomes de carbone et $R_2$ = $NHCOCH_3$, par une réaction mettant en jeu un dérivé de formule

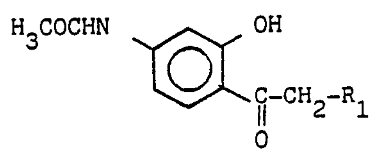

et le chlorure de l'acide paraméthoxycarbonylbenzoïque.

4. Procédé de préparation des composés selon la revendication 1 à partir des dérivés de formule (II)

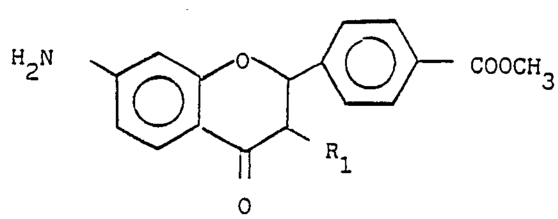

dans laquelle $R_1$ = H, OH, alkyle ramifié ou non contenant de 1 à 5 atomes de carbone, et par lequel la fonction $NH_2$ des composés de formule générale (II) est transformée en groupements amide d'acide carboxylique sulfonamide ou chlorosulfonyl ; le groupement chlorosulfonyl par réaction avec des amines conduit aux dérivés dans lesquels $R_2$ est $—SO_2N(CH_3)_2$, $—SO_2NHCH_3$, $—SO_2NHCH_2CH_2OH$ ou $SO_2NH_2$ et les acides de formule (I) sont obtenus à partir des esters de méthyl correspondant par des méthodes classiques de la chimie.

5. Procédé de préparation des composés de formule (II) selon la revendication 4 avec $R_1$ = alkyle ramifié ou non contenant de 1 à 5 atomes de carbone par une réaction entre un dérivé de formule

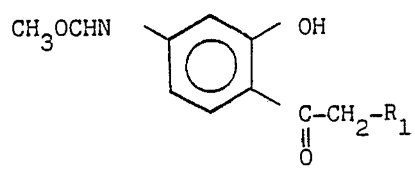

et le chlorure de l'acide paraméthoxycarbonylbenzoïque suivie d'une N-désacétylation du composé ainsi obtenu dans le méthanol en présence d'acide chlorhydrique gazeux.

6. Médicament caractérisé en ce que le principe actif est constitué par au moins un composé selon la revendication 1.

7. Médicament selon la revendication 6 utile en thérapeutique préventive et curative des complications du diabète.

8. Médicament selon la revendication 6 utile en tant que diurétique.

9. Composition pharmaceutique ou vétérinaire caractérisée en ce qu'elle contient à titre de principe actif au moins un composé selon la revendication 1 en association avec un véhicule pharmaceutique ou un excipient approprié.

## Claims

1. Compounds with the general formula

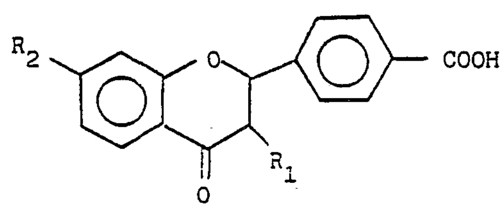

wherein $R_1$ = H, OH, $OCOCH_3$, $OSO_2CH_3$, branched or unbranched alkyl containing from 1 to 5 carbon atoms, $R_2 = NHCOR_3$, $NHCOCH_2NR_4R_5$, $NHSO_2CH_3$, $N(SO_2CH_3)_2$, $SO_2N(CH_3)_2$, $SO_2NHCH_3$, $SO_2NCH_3CH_2CH_2OH$, $SO_2NH_2$ with $R_3$, $R_4$ and $R_5$ = lower alkyl containing from 1 to 4 carbon atoms, $NR_4R_5$ may also form a piperidine, pyrrolidine, morpholine ring, as well as one of the following pharmaceutically-acceptable functions drived from the carboxylic acid function, that is the methyl ester, piperidine amide functions and the alkaline or alkaline-earth metal salts as well as the addition salts from pharmaceutically-acceptable inorganic or organic acids.

2. Method for preparing methyl esters from the compounds as defined in claim 1, wherein $R_1$ = branched or unbranched alkyl containing from 1 to 5 carbon atoms, and $R_2 = NHCOR_3$, $NHCOCH_2NR_4R_5$, $NHSO_2CH_3$, $N(SO_2CH_3)_2$, with $R_3$, $R_4$ and $R_5$ = lower alkyl containing from 1 to 4 carbon atoms or $NR_4R_5$ may also form a piperidine, pyrrolidine, morpholine ring, by a reaction bringing into play a derivative with as formula

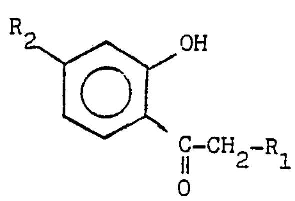

and paramethoxycarbonyl benzoic acid chloride.

3. Preparation method as defined in claim 2 for methyl esters from the compounds as defined in claim 1, wherein $R_1$ = branched or unbranched alkyl containing from 1 to 5 carbon atoms and $R_2 = NHCOCH_3$, by a reaction bringing into play a derivative with as formula

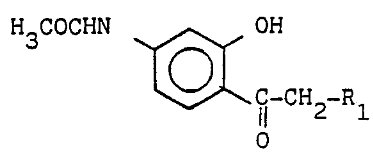

and paramethoxycarbonyl benzoic acid chloride.

4. Method for preparing compounds as defined in claim 1 from derivatives with as formula (II)

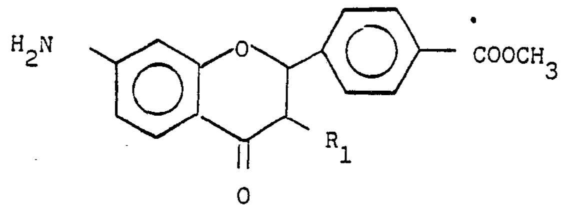

wherein $R_1$ = H, OH, branched or unbranched alkyl containing from 1 to 5 carbon atoms, and wherewith the $NH_2$ function from the compounds having the general formula (II) is converted into amide groups from carboxylic, sulfonamide or chlorosulfonyl acid, the chlorosulfonyl group leads by reacting with amines to those derivatives wherein $R_2$ is $—SO_2N(CH_3)_2$, $—SO_2NHCH_3$, $—SO_2NHCH_2CH_2OH$ or $—SO_2NH_2$ and the acids with formula (I) are obtained from corresponding methyl esters by means of conventional chemistry methods.

5. Method for preparing compounds with formula (II) as defined in claim 4, with $R_1$ = branched or unbranched alkyl containing from 1 to 5 carbon atoms by reacting a derivative with formula

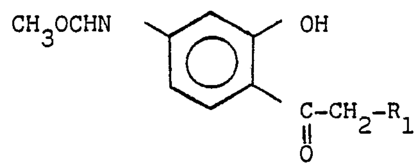

with paramethoxycarbonyl benzoic acid chloride, followed by N-unacetylating the resulting compound in methanol in the presence of gazeous hydrochloric acid.

6. Medicament, in which the active constituent is comprised of at least one compound as defined in claim 1.

7. Medicament as defined in claim 6, useful in preventive and curative therapy of diabetes involvements.

8. Medicament as defined in claim 6, useful as diuretic.

9. Pharmaceutical or veterinary composition, which contains as active constituent, at least one compound as defined in claim 1 associated with a suitable pharmaceutical medium or vehicle.

**Patentansprüche**

1. Verbindungen der allgemeinen Formel

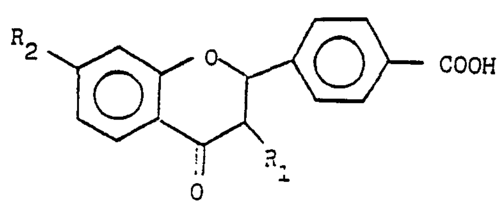

in der $R_1$ = H, OH, $OCOCH_3$, $OSO_2CH_3$, verzweigtes oder unverzweigtes Alkyl mit 1 bis 5 Kohlenstoffatomen, $R_2 = NHCOR_3$, $NHCOCH_2$, $NR_4R_5$, $NHSO_2CH_3$, $N(SO_2CH_3)_2$, $SO_2N(CH_3)_2$, $SO_2NHCH_3$, $SO_2NCH_3CH_2CH_2OH$, $SO_2,NH_2$ bedeuten, wobei $R_3$, $R_4$ und $R_5$ = niedriges Alkyl mit 1 bis 4 Kohlenstoffatomen bedeuten und $NR_4R_5$ ebenfalls einen Piperidin-, Pyrrolidin-, Morpholinring bilden können, sowie deren von der Carbonsäurefunktion abgeleitete, pharmazeutisch verträgliche funktionelle Derivate, nämlich Methylester und Piperidide sowie Alkali- und Erdalkalisalze, sowie Additionssalze derselben mit pharmazeutisch verträglichen Mineral- oder organischen Säuren.

2. Verfahren zur Herstellung der Methylester der Verbindungen nach Anspruch 1, bei denen $R_1$ = verzweigtes oder unverzweigtes Alkyl mit 1 bis 5 Kohlenstoffatomen und $R_2 = NHCOR_3$, $NHCOCH_2NR_4R_5$, $NHSO_2CH_3$, $N(SO_2CH_3)_2$ bedeuten, wobei $R_3$, $R_4$ und $R_5$ niedriges Alkyl mit 1 bis 4 Kohlenstoffatomen oder $NR_4R_5$ ebenfalls einen Piperidin-, Pyrrolidin-, Morpholinring bilden können, durch eine Reaktion von einem Derivat der Formel

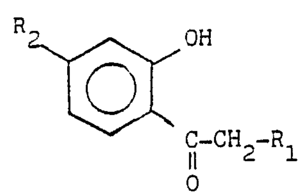

mit Paramethoxycarbonyl-benzoylchlorid.

3. Verfahren zur Herstellung nach Anspruch 2 von Methylestern der Verbindungen nach Anspruch 1, in denen $R_1$ = verzweigtes oder nicht-verzweigtes Alkyl mit 1 bis 5 Kohlenstoffatomen und $R_2$ = $NHCOCH_3$ ist, durch eine Reaktion eines Derivates der Formel

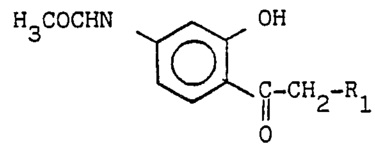

und Paramethoxycarbonyl-benzoylchlorid.

4. Verfahren zur Herstellung der Verbindungen nach Anspruch 1 aus Derivaten der Formel (II)

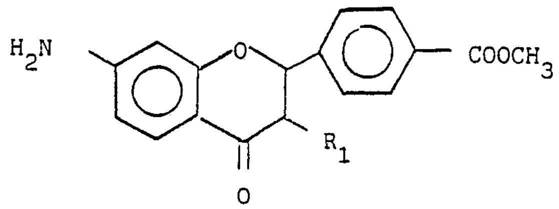

in der $R_1$ = H, OH, verzweigtes oder unverzweigtes Alkyl mit 1 bis 5 Kohlenstoffatomen bedeuten, bei dem die $NH_2$-Funktion der Verbindungen der allgemeinen Formel (II) in Carbonsäureamid-, Sulfonamid- oder Chlorsulfonylgruppen überführt wird und die Chlorsulfonylgruppe durch Umsetzung mit Aminen zu Derivaten führt, in denen $R_2$ $—SO_2N(CH_3)_2$, $—SO_2NHCH_3$, $—SO_2NHCH_2CH_2OH$ oder $SO_2NH_2$ ist, und die Säuren der Formel (I) mittels klassischer Methoden der Chemie aus den entsprechenden Methylestern erhalten werden.

5. Verfahren zur Herstellung der Verbindungen der Formel (II) nach Anspruch 4, wobei $R_1$ = verzweigtes oder unverzweigtes Alkyl mit 1 bis 5 Kohlenstoffatomen ist, durch eine Reaktion zwischen einem Derivat der Formel

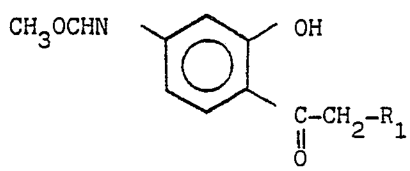

und Paramethoxycarbonyl-benzoylchlorid, gefolgt von einer N-Desacetylierung der so erhaltenen Verbindung in Methanol in Gegenwart von gasförmiger Salzsäure.

6. Arzneimittel, dadurch gekennzeichnet, daß der Wirkstoff aus mindestens einer Verbindung nach Anspruch 1 besteht.

7. Arzneimittel nach Anspruch 6 zur Verwendung in der vorbeugenden und kurativen Therapie von Komplikationen der Diabetes.

8. Medikament nach Anspruch 6 zur Verwendung als diuretisches Mittel.

9. Pharmazeutische oder veterinärmedizinische Zusammensetzung, dadurch gekennzeichnet, daß sie als Wirkstoff mindestens eine Verbindung nach Anspruch 1 zusammen mit einem pharmazeutischen Träger oder einem geeigneten Exzipienten enthält.